# EUROPÄISCHE PATENTSCHRIFT

(11) **EP 1 903 928 B1**
(45) Veröffentlichungstag und Bekanntmachung des Hinweises auf die Patenterteilung: **04.09.2019**
(21) Anmeldenummer: 06762667.1
(22) Anmeldetag: 18.07.2006
(51) Int. Cl.: A61B 3/032, A61B 3/036, A61B 3/06

(54) **VERFAHREN UND VORRICHTUNG ZUM PRÜFEN DES NACHTSEHENS**
METHOD AND DEVICE FOR TESTING SCOTOPIC VISION
PROCEDE ET DISPOSITIF POUR VERIFIER LA VISION SCOTOPIQUE

(30) Priorität: 19.07.2005 DE 102005034619
(43) Veröffentlichungstag der Anmeldung: 02.04.2008
(73) Patentinhaber: Carl Zeiss Vision GmbH, 73430 Aalen (DE)
(72) Erfinder: CABEZA GUILLEN, Jesús, Miguel, 73434 Aalen (DE); KRATZER, Timo, 73434 Aalen (DE)
(74) Vertreter: Witte, Weller & Partner Patentanwälte mbB
(86) Internationale Anmeldenummer: PCT/EP2006/007031
(87) Internationale Veröffentlichungsnummer: WO 2007/009736

(56) Entgegenhaltungen:
- WO-A-02/078529
- CN-U- 2 087 946
- DE-U- 1 927 704
- DE-U1- 20 310 162
- GB-A- 982 874
- GB-A- 2 355 540
- US-A- 5 471 262
- US-A- 5 988 814
- US-A1- 2002 044 259

## Beschreibung

Die Erfindung betrifft ein Verfahren zum Prüfen des Nachtsehens, bei dem einem Probanden mit geöffneter Pupille, vorzugsweise in einem abgedunkelten Raum, mindestens ein Sehzeichen aus einer Entfernung von mehreren Metern angeboten wird.

Die Erfindung betrifft ferner eine Vorrichtung zum Prüfen des Nachtsehens, mit Mitteln zum Öffnen der Pupille eines Probanden, vorzugsweise mit einem abgedunkelten Raum, mit einer Untersuchungsposition für den Probanden, und mit einer Präsentationsvorrichtung für Sehzeichen, die in einem Abstand von mehreren Metern von der Untersuchungsposition angeordnet ist.

Ein Verfahren und eine Vorrichtung der vorstehend genannten Art sind aus der DE 30 03 588 C2 bekannt.

Die refraktiven Eigenschaften eines Auges hängen unter anderem vom Pupillendurchmesser ab. Dies bedeutet, dass eine Refraktionsmessung, die unter normalen Beleuchtungsbedingungen durchgeführt wurde, bei schwacher Beleuchtung nicht mehr gilt, weil die Pupille sich vergrößert hat. Wenn also eine Person eine Brille besitzt, die bei Tageslicht optimiert ist, dann kann die Sehschärfe in der Dämmerung und insbesondere bei Nachtfahrten in einem Fahrzeug herabgesetzt sein, weil die Brille für diese Beleuchtungsverhältnisse nicht optimiert ist. So kommt es vor, dass Personen tagsüber gut sehen, sei es mit oder ohne Brille, nachts hingegen fast blind sind, weil die Tagesrefraktion ihrer Augen vollständig anders als ihre Nachtrefraktion ist.

Im Stand der Technik sind bereits verschiedene Verfahren und Vorrichtungen vorgeschlagen geworden, um das Sehverhalten in der Dämmerung und in der Dunkelheit zu prüfen. Dazu gehören auch andere Phänomene, wie zum Beispiel die Adaptation, das heißt die Fähigkeit des Auges sich mehr oder weniger schnell von hellen Umgebungsbedingungen an dunkle Bedingungen anzupassen.

Zur Messung des Seheindrucks, speziell der Sehschärfe eines Auges sind sowohl subjektive wie objektive Vorgehensweisen bekannt. Der Seheindruck umfasst dabei die gesamte Wahrnehmung des Probanden, also auch eventuelle Beschränkungen seines Sehfeldes am Rand (Tunnelblick) oder in Gestalt von flächigen Ausfällen im Sehfeld (Skotome). Die Sehschärfe betrifft, unabhängig von derartigen Einschränkungen, nur das Scharfsehen, also die Erkennung von Details

Beim subjektive Ansatz werden dem Probanden bestimmte Sehzeichen präsentiert, die er durch eine Probebrille betrachtet, in die der Untersucher nacheinander verschiedene Probe-Brillengläser einsetzt. Der Proband teilt dann dem Untersucher mit, bei welchem Probe-Brillenglas er das Sehzeichen am besten, speziell am schärfsten erkennen kann. Es fließt also die subjektive Beurteilung des Probanden in die Untersuchung ein.

Beim objektiven Ansatz hingegen wird z.B. die Refraktion des Auges ohne Interaktion mit dem Probanden vermessen, beispielsweise mit einem Wellenfront-Analysegerät. Diese Methode führt zwar zu einem objektiv optimalen Ergebnis, das sich in der Praxis allerdings nicht immer mit dem subjektiven Eindruck des Probanden deckt. Daher werden beide Methoden häufig nebeneinander eingesetzt, um zu einem optimalen Ergebnis zu gelangen.

Bei Vorrichtungen zum Messen der Sehfähigkeit eines Auges unterscheidet man zwischen solchen, bei denen der Proband frei in einem Raum sitzt und Sehzeichen betrachtet, die in einer Entfernung von mehreren Metern erscheinen, ferner solchen, die als kastenartige Tischgeräte ausgebildet sind und in die der Proband über zwei Okulare hineinsieht, und schließlich solchen, die dem Probanden als Brille oder Helm aufgesetzt werden. Die beiden letztgenannten Vorrichtungen haben den Nachteil, dass sie zu einer sog. Geräte-Kurzsichtigkeit (device myopia) führen können, weil der Proband nicht unter normalen Bedingungen sieht.

Aus der eingangs erwähnten DE 30 03 588 C2 ist bekannt, das Dämmerungssehen, die Blendfestigkeit und die Adaptationsfähigkeit mittels einer Anordnung zu prüfen, bei der bestimmte Sehzeichen mit vorbestimmter Helligkeit und Umgebungshelligkeit auf einen Schirm projiziert werden. Der Proband befindet sich dabei in einem Abstand von mindestens drei Metern vom Schirm. Die Sehzeichen sind dabei von bekannter Art, insbesondere sog. Landolt-Ringe. Eine präzise Untersuchung und Bestimmung der Nachtsehschärfe ist damit nicht möglich.

Aus der US 3,328,113 ist ein Gerät zum Testen des Nachtssichtvermögens bekannt. Bei diesem bekannten Gerät werden dem Probanden periphere Lichtpunkte als Sehzeichen angeboten. Die Lichtpunkte werden mit einer Blinklichtquelle und einer davor angeordneten, drehbaren Blendenscheibe in Verbindung mit Radialschlitzen in der Frontplatte des Gerätes erzeugt. Die Blendenscheibe ist mit Öffnungen unterschiedlichen Durchmessers im Bereich zwischen 0,6 und 14 mm und unterschiedlichem Abstand von der Drehachse der Blendenscheibe versehen. Auf diese Weise lassen sich durch Drehung der unmittelbar hinter den Radialschlitzen angeordneten Blendenscheibe Lichtpunkte unterschiedlicher Größe und unterschiedlichen Abstandes von der Drehachse anbieten. Eine Kinnstütze für den Probanden befindet sich ca. 40 cm vor dem Gerät. Mit diesem bekannten Gerät lässt sich daher ebenfalls nur das Sehvermögen bei Dunkelheit testen. Eine Messung der Sehschärfe ist nicht vorgesehen.

Aus dem Dokument US 2002/0044259 A1 ist eine Vorrichtung zum Prüfen des skotopischen Sehens bekannt, bei der ein Proband im Abstand von zwei Metern von einer Sehzeichen-Lichtquelle entfernt sitzt, die aus einem zentralen Lichtpunkt von 10 mm Durchmesser und mehreren darum herum angeordneten Lichtpunkten von 1 mm Durchmesser besteht. Der große, zentrale Lichtpunkt ist immer eingeschaltet und die kleinen Lichtpunkte werden in vorbestimmter Weise nacheinander eingeschaltet. Der Proband soll einen Taster betätigen, wenn er das Erscheinen der kleinen Lichtpunkte bemerkt.

Aus dem Dokument GB 982 874 A ist es bekannt, einem Probanden ein Bild anzubieten, das aus mehreren Matchbox-Autos besteht, die sich nebeneinander im gestuften Abstand zum Probanden befinden und sich dem Probanden dann mit unterschiedlicher Geschwindigkeit nähern, derart, dass sie einander überholen. Der Proband soll ein Signal geben, wenn die Autos nach seinem Eindruck auf gleicher Höhe sind.

Das Dokument WO 02/078529 A1 beschreibt eine Vorrichtung, bei der ein Proband einen Bildschirm sieht, auf dem ihm Sehzeichen, insbesondere eine Zahl oder ein Buchstabe oder ein alltäglicher Gegenstand, angeboten werden. Dieses Sehzeichen füllt den Bildschirm aus. Der Proband soll angeben, ob er das Sehzeichen in seiner Bedeutung erkannt hat.

In dem Dokument CN 2087946 U ist ein weiteres Gerät zum Testen des Sehens geziegt, das des Weiteren mehrere lichtemittierende Dioden aufweist.

Daneben sind im Stand der Technik auch Tischgeräte beschrieben (DE 23 21 570; EP 0 830 838 A2; JP 2001-087225 A) sowie helm- oder brillenartige Anordnungen (DE 43 26 760 A1; DE 197 29 102 C2), die u.a. zum Prüfen des Dämmerungssehens dienen, dabei jedoch herkömmliche Sehzeichen verwenden sowie teilweise Blendeinrichtungen, um die Adaptationsfähigkeit zu testen.

Der Erfindung liegt daher die Aufgabe zugrunde, ein Verfahren und eine Vorrichtung der eingangs genannten Art dahingehend weiterzubilden, dass die vorgenannten Nachteile vermieden werden. Insbesondere soll es möglich werden, die Nachtsehschärfe erheblich genauer als bisher prüfen zu können. Dies ermöglicht es, den betroffenen Personen Sehhilfen zur Verfügung zu stellen, mit denen sie in der Dämmerung und in der Nacht erheblich schärfer sehen können. Für Kraftfahrer stellt dies unter den genannten Umgebungsbedingungen einen deutlichen Sicherheitsgewinn dar.

Daher wird eine Ausgestaltung der Erfindung gemäß der unabhängigen Ansprüche bereitgestellt.

Die der Erfindung zugrunde liegende Aufgabe wird auf diese Weise vollkommen gelöst.

Die Erfindung löst sich nämlich grundsätzlich von der herkömmlichen Vorgehensweise, die mit flächigen Sehzeichen arbeitet. Bei diesen Sehzeichen ist immer eine gewisse Hintergrundbeleuchtung vorhanden, die eine genaue Bestimmung einer optimalen Korrektur der Sehschärfe verhindert, wenn dem Probanden eine Probebrille aufgesetzt und nacheinander unterschiedliche Probe-Brillengläser eingesetzt werden.

Erfindungsgemäß wird vielmehr eine möglichst punktförmige Lichtquelle als Sehzeichen verwendet. Der Proband sieht diesen Lichtpunkt je nach Fehlsichtigkeit mit vergrößertem kreis- oder ellipsenförmigem Umriss und - vor allem - mit seitlichen abfallenden Flanken. Diese Flanken rühren von der sog. Punktverbreiterungsfunktion (point spread function PSF) her. Die PSF bewirkt bei einem idealen, d.h. aberrationsfreien und auf unendlichen Abstand eingestellten System, dass die Lichtverteilung in der Abbildung einer unendlich entfernten punktförmigen Lichtquelle in radialer Richtung vom Zentrum einen Intensitätsverlauf in Form einer Bessel-Funktion erster Ordnung mit einer schmalen Spitze und seitlich abfallenden Flanken hat. Diese Flanken sind für einen Probanden nur bei nahezu vollständiger Dunkelheit zu erkennen. Bereits bei verhältnismäßig schwacher Umgebungshelligkeit verschwinden sie in dieser und sind nicht mehr erkennbar.

Die Messung in einem dunklen Raum hat den Vorteil, dass die Pupille des Probanden weit geöffnet ist, was beispielsweise den Bedingungen einer Nachtfahrt weit mehr entspricht als die Verhältnisse bei herkömmlichen Messungen, bei denen infolge des vorhandenen Restlichtes die Pupillen nicht vollständig geöffnet sind. Bei der erfindungsgemäßen Vorgehensweise tritt nämlich wegen der punktförmigen Lichtquelle nur eine deutlich geringere Lichtintensität in das Auge ein, verglichen mit herkömmlichen Sehzeichentafeln mit beleuchtetem Hintergrund. Dies schließt natürlich nicht aus, dass im Rahmen der vorliegenden Erfindung auch eine Blendung simuliert werden kann.

Wenn man daher entsprechend der vorliegenden Erfindung vorgeht, kann man einem Probanden nacheinander unterschiedliche Probe-Brillengläser anbieten und der Proband ist im Stande, sehr genau anzugeben, bei welchen Probe-Brillengläsern er eine optimale Sehschärfe hat, nämlich dann, wenn die von ihm gesehenen Spitze möglichst schmal und die seitlich abfallenden Flanken minimal sind.

Im Stand der Technik sind ophthalmologische Untersuchungsverfahren- und Vorrichtungen bekannt, die mit punktförmigen Lichtquellen arbeiten.

In der US 6,540,356 B1 ist beispielsweise ein sog. Tscherning'sches Aberrometer beschrieben. Bei dieser Vorrichtung wird im oben erläuterten Sinne objektiv gemessen, d.h. ohne Mitwirkung des Probanden. Es werden mittels Leuchtdioden ein oder mehrere schmale, kollimierte Strahlen erzeugt und in das Auge des Probanden projiziert. Die Ablenkung der Strahlen auf die Ebene der Retina wird gemessen. Im Gegensatz dazu wird im Rahmen der vorliegenden Erfindung eine punktförmige Lichtquelle verwendet, die das Auge als sphärische Wellen erfasst. Eine Ablenkung wird nicht gemessen.

Bei anderen bekannten Vorrichtungen (JP 05-285107 A; JP 2003-093244 A; US 5 080 478; DE 197 29 102 C2) werden zwar Leuchtdioden als punktförmige Lichtquellen eingesetzt, die dabei erzeugten Lichtpunkte dienen jedoch als Fixierungstarget oder als Stimuli, um die Adaptationsfähigkeit zu testen.

Der Lichtpunkt bzw. der von der Lichtquelle ausgesendete Lichtstrahl weist einen Durchmesser von weniger als 1 mm auf.

Diese Dimensionierung hat sich in der Praxis als optimaler Kompromiss zwischen einem möglichst kleinen Durchmesser, d.h. einer möglichst punktförmigen Lichtquelle einerseits und einer möglichst großen Helligkeit, die einen gewissen Durchmesser erfordert, erwiesen. Bevorzugt wird ein Durchmesser von etwa 0,9 mm verwendet, der bei einem Abstand von ca. 6 m für Probanden mit einer Sehschärfe von 2 (40/20) geeignet ist.

Die mindestens eine punktförmige Lichtquelle ist als Leuchtdiode ausgebildet.

Diese Maßnahme hat den Vorteil, dass erprobte Bauelemente eingesetzt werden können, die einen niedrigen Energieverbrauch aufweisen und deren Intensität in einfacher Weise einstellbar ist.

Auch kann die mindestens eine punktförmige Lichtquelle als Leuchtpunkt eines Bildschirms ausgebildet sein.

Diese Maßnahme hat den Vorteil, dass zur Darstellung des Lichtpunktes eine Ebene eine Ebene zur Verfügung steht, so dass einerseits die Position des Lichtpunktes in dieser Ebene frei vorgebbar ist, andererseits aber auch bestimmte Muster von Lichtpunkten dargestellt werden können, beispielsweise die oben bereits erwähnten Paare und Reihen, und zwar in unterschiedlicher Gestalt und zeitlicher Reihenfolge.

Hierbei ist erfindungsgemäß bevorzugt, wenn der Bildschirm ein LED-Bildschirm, insbesondere ein OLED-Bildschirm ist.

Diese Maßnahme hat den Vorteil, dass große Flächen mit hoher Intensität der Leuchtpunkte realisiert werden können.

Schließlich ist noch ein Ausführungsbeispiel der erfindungsgemäßen Vorrichtung bevorzugt, bei dem der Untersuchungsposition ein Magazin von Proben-Brillengläsern für eine vom Probanden getragene Probebrille zugeordnet ist.

Diese Maßnahme hat den Vorteil, dass eine Untersuchungsperson dem Probanden in an sich bekannter Weise verschiedene Probe-Brillengläser anbieten kann, um auf diese Weise die für den Probanden optimalen Gläser zu ermitteln.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Figur 1:: eine äußerst schematisierte Seitenansicht eines Ausführungsbeispiels einer erfindungsgemäßen Vorrichtung;
- Figur 2:: eine Vorderansicht einer in der Vorrichtung gemäß Figur 1 verwendeten Präsentationsvorrichtung;
- Figur 3:: ein erstes Ausführungsbeispiel eines erfindungsgemäß verwendeten Sehzeichens in Gestalt eines Lichtpunktes;
- Figur 4:: das punktförmige Sehzeichen aus Figur 3, so wie es von einem fehlsichtigen Probanden wahrgenommen wird;
- Figur 5:: ein Beispiel eines verwendeten Sehzeichens in Gestalt eines Paares von Lichtpunkten zum besseren Verständnis der Erfindung;
- Figur 6:: ein Beispiel eines verwendeten Sehzeichens in Gestalt mehrerer Paare von Lichtpunkten zum besseren Verständnis der Erfindung; und
- Figur 7:: ein Beispiel eines verwendeten Sehzeichens in Gestalt mehrerer Reihen von Lichtpunkten zum besseren Verständnis der Erfindung.

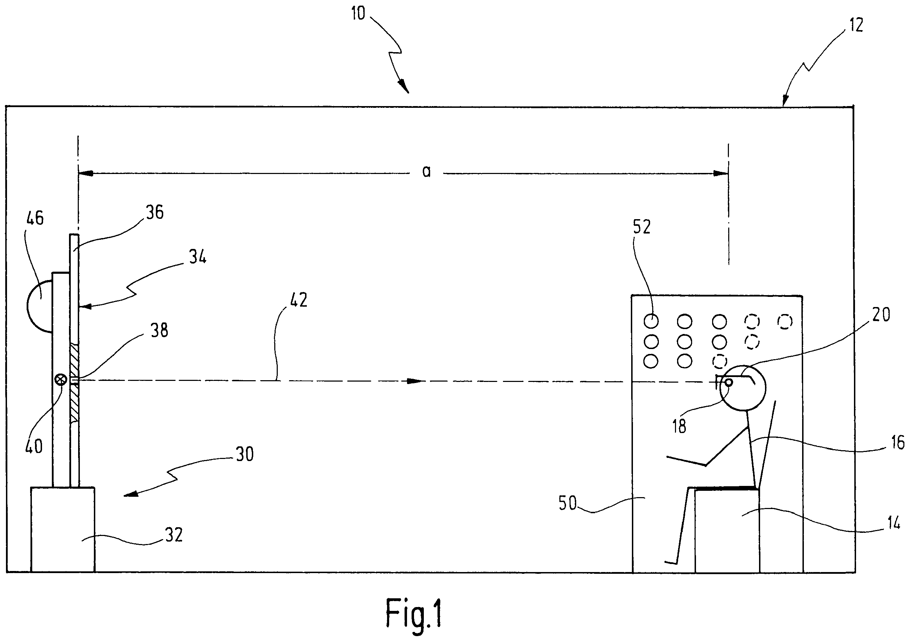

In Figur 1 bezeichnet 10 als ganzes ein Ausführungsbeispiel einer erfindungsgemäßen Vorrichtung zum Prüfen der Nachtssehschärfe, mit der das erfindungsgemäße Verfahren ausführbar ist.

Für die vorliegende Erfindung ist es wichtig, dass die Pupillen des Probanden geöffnet sind. Die Vorrichtung 10 umfasst hierzu einen vorzugsweise abgedunkelten Raum 12. Unter "abgedunkelt" ist dabei zu verstehen, dass der Raum nach außen lichtdicht ausgeführt ist und nach Abschalten einer gesamthaften oder punktuellen Orientierungsbeleuchtung vollständig dunkel ist. Dies schließt natürlich nicht aus, dass auch eine bestimmte Helligkeit in dem Raum 12 eingestellt werden kann und die Pupillen durch Gabe eines Mittels (Atropintropfen) künstlich geöffnet sind. Die bevorzugte Betriebsart ist jedoch die vollständige Dunkelheit, weil der Proband dann nach einer bestimmten Adaptationszeit vollständig geöffnete Pupillen hat.

In dem Raum 12 befindet sich in Figur 1 am rechten Ende eine Untersuchungsposition 14, im einfachsten Fall ein Stuhl für einen Probanden 16. In Figur 1 ist ein Auge 18 des Probanden 16 eingezeichnet. Der Proband 16 trägt eine Probebrille 20 üblicher Bauart, in die von einer Untersuchungsperson verschiedene Probe-Brillengläser eingesetzt werden können.

An dem in Figur 1 linken Ende des Raumes 12 befindet sich eine Präsentationsvorrichtung 30 für Sehzeichen. Die Präsentationseinrichtung 30 ist von der Untersuchungsposition 14 in einem Abstand a angeordnet, der so groß wie möglich sein soll und in der Praxis bei mehreren Metern, beispielsweise bei etwa 6 m liegt.

Die Präsentationsvorrichtung 30 weist einen Sockel 32 auf, der eine Sehzeichenquelle 34 trägt. Die Sehzeichenquelle 34 kann in unterschiedlicher Weise ausgebildet sein.

In dem in Figur 1 dargestellten Beispiel besteht die Sehzeichenquelle 34 aus einer Blende 36 mit einer zentralen Öffnung 38. Hinter der Öffnung 38 befindet sich ein Leuchtmittel 40.

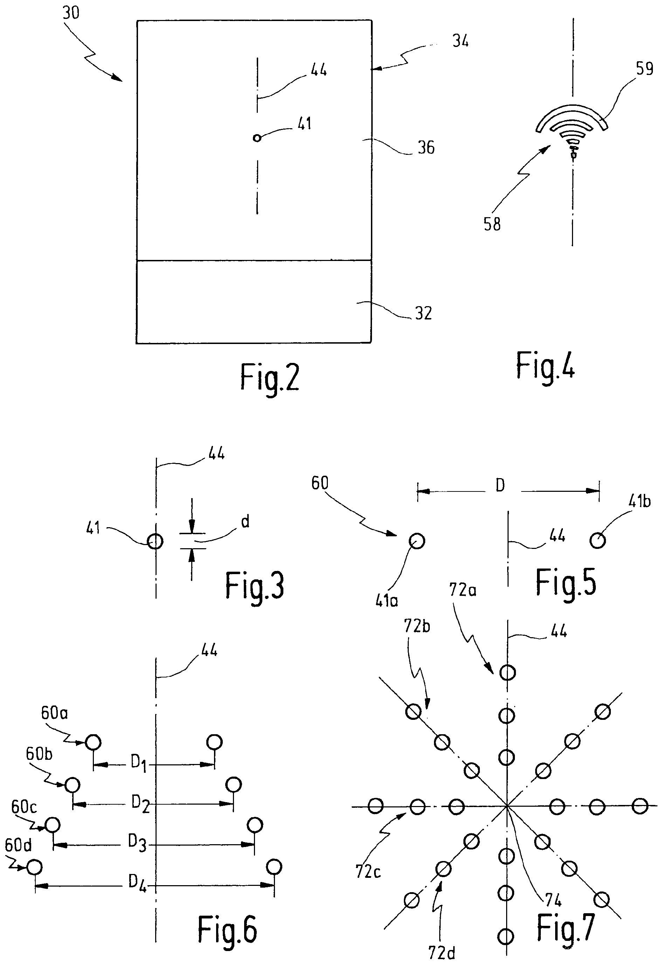

Auf diese Weise wird, wie die Vorderansicht der Präsentationsvorrichtung 30 in Figur 2 zeigt, eine punktförmige Lichtquelle 41 bzw. ein Lichtpunkt als Sehzeichen dargestellt. Die punktförmige Lichtquelle 41 sendet einen Lichtstrahl 42 aus, der auf die Untersuchungsposition 14 gerichtet ist und in das Auge 18 des Probanden 16 fällt.

Die Figuren 2 und 3 zeigen, dass die punktförmige Lichtquelle 41 bzw. der Lichtpunkt sich auf einer Achse 44 befinden. Der Lichtpunkt 41 hat einen Durchmesser d, der kleiner als 1 mm ist. Bevorzugt ist ein Durchmesser von 0,9 mm. Dieser Wert ist bei einem Abstand a von ca. 6 m geeignet für Probanden 16 mit einer Sehschärfe von 2 (40/20). Es können auch weitere Sehzeichen relativ zu der Achse 44 erzeugt werden, wie weiter unten anhand der Figuren 5 bis 7 noch erläutert werden wird.

In dem Raum 12 ist noch eine vorzugsweise indirekte Beleuchtung 46 vorgesehen. Diese kann auf der Rückseite der Präsentationsvorrichtung 30 angeordnet und derart einstellbar sein, dass in dem Raum 12 zu Mess- oder Orientierungszwecken eine gewisse Helligkeit erzeugt wird. Die Beleuchtung 46 kann auch so ausgebildet und angeordnet sein, dass sie zum kurzzeitigen Blenden des Probanden 16 geeignet ist, so dass auch Blendsituationen simuliert werden können, wie sie bei Nachtfahrten auftreten.

Ferner ist in dem Raum 12 im Bereich der Untersuchungsposition 14 noch ein Magazin 50 für Probe-Brillengläser 52 angeordnet, damit eine Untersuchungsperson in der bereits angesprochenen Weise nacheinander verschiedene Probe-Brillengläser 52 in die vom Probanden 16 getragene Probebrille 20 einsetzen kann. Auch dort kann eine Orientierungsbeleuchtung vorgesehen sein (nicht dargestellt).

Figur 4 zeigt ein Sehzeichen 58, so wie es von einem fehlsichtigen Probanden 16 wahrgenommen wird. Das wahrgenommene Sehzeichen 58 hat eine kreis- bzw. ellipsenförmige Kontur und läuft seitlich in abfallenden Flanken 59 aus. Diese Form des wahrgenommenen Sehzeichens 58 beruht auf der bereits eingangs erwähnten sog. Punktverbreiterungsfunktion (point spread function PSF) .

Wie erwähnt, bewirkt die PSF bei einem idealen, d.h. aberrationsfreien und auf unendlichen Abstand eingestellten System, dass die Lichtverteilung in der Abbildung der im Abstand a befindlichen punktförmigen (Durchmesser d) Lichtquelle 41 in radialer Richtung vom Zentrum einen Intensitätsverlauf in Form einer Bessel-Funktion erster Ordnung mit einer schmalen Spitze und seitlich abfallenden Flanken hat. Im Falle einer Kurz- oder Weitsichtigkeit des Probanden 16 reagiert die PSF sehr empfindlich und erhält anstatt einer schmalen Spitze die Form eines Kreises, dessen Durchmesser vom Ausmaß dieser Fehlsichtigkeit abhängt, im Falle eines Astigmatismus hat er die Form einer Ellipse.

In jedem Fall bleiben die seitlichen abfallenden Flanken erhalten, deren vom Probanden 16 wahrgenommene Größe ein Maß dafür ist, in welchem Ausmaß die Fehlsichtigkeit durch Einsetzen entsprechender Probe-Brillengläser 52 in die Probebrille 20 bereits korrigiert wurde, und zwar bei in der umgebenden Dunkelheit weit geöffneter Pupille. Bei zunehmender Korrektur verschmälert sich daher das wahrgenommene Sehzeichen 58 in Figur 4, bis es im Idealfall die Gestalt einer schmalen Spitze mit minimalen seitlichen Flanken 59 annimmt. Hierin liegt ein besonderer Vorteil der Erfindung, weil die Messung in einem dunklen Raum 12 dazu führt, dass der Proband 16 die seitlich abfallenden Flanken 59 sehr gut erkennen kann, was bereits bei geringem Umgebungslicht nicht mehr möglich ist.

In Figur 5 ist lediglich beispielhaft dargestellt, dass anstatt eines einzigen Lichtpunktes auch ein Paar 60 von Lichtpunkten 41a und 41b verwendet wird, die sich beidseits der Achse 44 in einem vorgegebenen Abstand D zueinander befinden. Der Proband 16 kann in diesem Falle aussagen, ob er tatsächlich zwei Lichtpunkte 41a und 41 b oder nur einen Lichtpunkt sieht.

In Figur 6 ist lediglich ein Beispiel dargestellt, bei der mehrere Paare 60a, 60b, 60c und 60d parallel zueinander entlang der Achse 44 angeordnet sind, wobei die Paare 60a, 60b, 60c und 60d zunehmende Abstände D₁, D₂, D₃ und D₄ aufweisen. Derartige Muster erlauben es, ähnlich wie bei bekannten Sehzeichen in Form eines Landolt'sehen Ringes, die Sehschärfe besonders gut zu erfassen.

Schließlich zeigt Figur 7 noch ein Beispiel mit vier Reihen 72a, 72b, 72c, und 72d von jeweils sechs Lichtpunkten, die im allgemeinen Fall unterschiedlich ausgerichtet sind und sich im dargestellten Beispiel unter gleichen Winkeln von 45° in einem Mittelpunkt 74 schneiden. Diese Anordnung gestattet es, zusätzlich einen Astigmatismus des Probanden 16 zu untersuchen.

Wie bereits weiter oben erwähnt wurde, kommen für die punktförmige Lichtquelle 41 mehrere praktische Bauformen in Betracht.

Neben der in Figur 1 dargestellten hinterleuchteten Blendenanordnung kann bevorzugt auch mit Leuchtdioden (LED) gearbeitet werden. Leuchtdioden sind heutzutage in sehr kleinen Abmessungen erhältlich (Durchmesser bis 0,200 µm einschließlich Sockel). Ihre Helligkeit kann in einfacher weise durch Einstellen des Versorgungsstromes geregelt werden. Sie benötigen nur eine geringe Versorgungsspannung von typischerweise 5V und haben einen hohen Wirkungsgrad.

Insbesondere für die Darstellung von Mustern von Lichtpunkten (Figuren 5 bis 7) sind flächenhafte Displays hoher Leuchtstärke geeignet. Diese Displays können LED-Displays sein, insbesondere OLED-Displays, die organische Leuchtdioden verwenden und besonders große Flächen haben können. Alternativ kann auch ein von hinten flächig erleuchtetes Display verwendet werden, das auf der Vorderseite mit einer steuerbaren Blendeneinreichtung versehen ist, beispielsweise einer Flüssigkristallanordnung (LCD), die es gestattet, durch selektive Abdeckung die einzelnen Pixel des Displays optisch ein- und auszuschalten.

## Patentansprüche

1. Verfahren zum Prüfen des Nachtsehens, bei dem einem Probanden (16) mit geöffneter Pupille in einem abgedunkelten Raum (12) ein Sehzeichen aus einer Entfernung von mehreren Metern angeboten wird, **dadurch gekennzeichnet, dass** das Sehzeichen als ein Lichtpunkt (41) ausgebildet ist, und dass die Sehschärfe des Probanden (16) mit Hilfe des Sehzeichens ermittelt wird, wobei der Lichtpunkt (41) als Leuchtdiode ausgebildet ist oder als Leuchtpunkt eines LED-Bildschirms ausgebildet ist, und wobei der Lichtpunkt (41) einen Durchmesser von weniger als 1 mm aufweist.

2. Vorrichtung zum Prüfen des Nachtsehens, mit einem abgedunkelten Raum (12), mit einer Untersuchungsposition (14) für den Probanden (16), und mit einer Präsentationsvorrichtung (30) für ein Sehzeichen, die in einem Abstand von mehreren Metern von der Untersuchungsposition (14) angeordnet ist, **dadurch gekennzeichnet, dass** die Präsentationsvorrichtung (30) eine punktförmige Lichtquelle (41) enthält, die das Sehzeichen ausbildet, dass der Lichtstrahl (42) der punktförmigen Lichtquelle (41) auf die Untersuchungsposition (14) gerichtet ist, und dass Mittel zum Ermitteln der Sehschärfe des Probanden anhand des Sehzeichens vorgesehen sind, wobei die eine punktförmige Lichtquelle (41) als Leuchtdiode ausgebildet ist oder als Leuchtpunkt eines LED-Bildschirms ausgebildet ist, und wobei der von der Lichtquelle (41) ausgesendete Lichtstrahl (42) einen Durchmesser von weniger als 1 mm aufweist.

3. Vorrichtung nach Anspruch 2, **dadurch gekennzeichnet, dass** der Untersuchungsposition (14) ein Magazin (50) von Proben-Brillengläsern (52) für eine vom Probanden (16) getragene Probebrille (20) zugeordnet ist.

## Claims

1. Method for checking scotopic vision, in which an optotype is presented to a subject (16) with an open pupil from a distance of several metres in a darkened room (12), **characterized in that** the optotype is embodied as a light spot (41) and **in that** the visual acuity of the subject (16) is ascertained with the aid of the optotype, wherein the light spot (41) is embodied as a light-emitting diode or embodied as an illuminated dot of an LED screen and wherein the light spot (41) has a diameter of less than 1 mm.

2. Apparatus for checking scotopic vision, comprising a darkened room (12), comprising an examination position (14) for the subject (16) and comprising a presentation apparatus (30) for an optotype, said presentation apparatus being arranged at a distance of several metres from the examination position (14), **characterized in that** the presentation apparatus (30) contains a punctiform light source (41) that forms the optotype, **in that** the light beam (42) of the punctiform light source (41) is directed to the examination position (14) and **in that** means for ascertaining the visual acuity of the subject on the basis of the optotype are provided, wherein the one punctiform light source (41) is embodied as a light-emitting diode or embodied as an illuminated dot of an LED screen and wherein the light beam (42) emitted by the light source (41) has a diameter of less than 1 mm.

3. Apparatus according to Claim 2, **characterized in that** a cartridge (50) of test spectacle lenses (52) for test spectacles (20) worn by the subject (16) are assigned to the examination position (14) .

## Revendications

1. Procédé de contrôle de la vision nocturne, selon lequel un optotype est présenté à un sujet (16) à la pupille ouverte depuis une distance de plusieurs mètres, **caractérisé en ce que** l'optotype est réalisé sous la forme d'un point lumineux (41) et **en ce que** l'acuité visuelle du sujet (16) est déterminée à l'aide de l'optotype, le point lumineux (41) étant réalisé sous la forme d'une diode électroluminescente ou sous la forme d'un point éclairé d'un écran à LED, et le point lumineux (41) possédant un diamètre inférieur à 1 mm.

2. Dispositif de contrôle de la vision nocturne, comprenant un espace obscurci (12), comprenant une position d'examen (14) pour le sujet (16) et comprenant un dispositif de présentation (30) pour un optotype, lequel est disposé à un écart de plusieurs mètres de la position d'examen (14), **caractérisé en ce que** le dispositif de présentation (30) contient une source de lumière ponctuelle (41) qui représente l'optotype, **en ce que** le rayon lumineux (42) de la source de lumière ponctuelle (41) est dirigé sur la position d'examen (14) et **en ce que** des moyens sont présents pour déterminer l'acuité visuelle du sujet à l'aide de l'optotype, ladite source de lumière ponctuelle (41) étant réalisée sous la forme d'une diode électroluminescente ou sous la forme d'un point éclairé d'un écran à LED, et le rayon lumineux (42) émanant de la source de lumière (41) possédant un diamètre inférieur à 1 mm.

3. Dispositif selon la revendication 2, **caractérisé en ce qu'**un chargeur (50) de verres de lunette d'essai (52) pour des lunettes d'essai (20) portées par le sujet (16) est associé à la position d'examen (14).
